# EUROPEAN PATENT SPECIFICATION

(11) **EP 1 351 656 B1**
(45) Date of publication and mention of the grant of the patent: **01.04.2009**
(21) Application number: 01270309.6
(22) Date of filing: 13.12.2001
(51) Int. Cl.: A61K 8/92, A61K 8/97, A61Q 5/10

(54) **HENNA PRODUCT**
HENNAPRODUKT
PRODUIT D'HENNE

(30) Priority: 15.12.2000 GB 0030705
(43) Date of publication of application: 15.10.2003
(73) Proprietor: Cosmetic Warriors, Poole Dorset BH15 1AB (GB)
(72) Inventor: CONSTANTINE, Mark, Poole, Dorset BH15 1AB (GB); CONSTANTINE, Margaret, Poole, Dorset BH15 1AB (GB); AMBROSEN, Helen, Poole, Dorset BH15 AB (GB)
(74) Representative: Kenyon, Sarah Elizabeth
(86) International application number: PCT/GB2001/005525
(87) International publication number: WO 2002/047634

(56) References cited:
- DE-A- 3 609 962
- US-A- 5 997 889
- US-A- 6 139 853

## Description

The present invention relates to products, preferably cosmetic products, used for dyeing. Although the invention is primarily for use on hair it may also be used on skin and other materials.

Henna has traditionally been sold as a powder and occasionally as a paste, and as a colouring in hair rinses, fabric dyes, etc.

Hair treatments and products, particularly cosmetic products, used for dyeing are conventionally supplied in containers made of a rigid or semi-rigid plastic material or of foil in the form of sachets. The container adds significantly to the costs of the product and environmental pollution is caused by disposal of the containers. Henna has been sold in containers of the aforementioned type.

Document DE3609962A discloses a hair colourant in the form of a solid tablet and containing henna.

Document US5997889 discloses a hand and body cream which has the form of a creamy solid and which contains cocoa butter.

Document US 6 139 853 discloses a powder or granular hair colorant comprising henna.

According to a first aspect of the present invention there is provided a product as defined in claim 1.

According to a second aspect of the present invention there is provided a method as defined in claim 15.

Preferably the product is formed of 40% to 90% henna by weight and 10% to 60% cocoa butter by weight. More preferably the product is formed of 60% to 80% henna by weight and 20% to 40% cocoa butter by weight. Most preferably the product is formed of 70% henna by weight and 30% cocoa butter by weight.

Additives may be provided, such as a surfactant, a conditioning agent, a fragrance, a herb, a fruit extract, a vegetable, a natural wax or butter, a synthetic wax, a humectant, glycerine, a vegetable oil.

Embodiments of the present invention will now be described in more detail by way of further example only.

In accordance with the present invention it has been determined that the simple mixture of henna and cocoa butter produces a very stable solid form product. The product can be very easily formed into many useful and/or novelty shapes. These can be displayed to form an attractive feature of the product in a shop.

The solid form of the product according to the invention enables a beneficial reduction in costs and avoidance of environmental pollution to be achieved by avoiding the necessity of a container of the aforementioned type. Further, by virtue of being in stolid form, the product can accept imprints during a moulding process.

Cocoa butter gives the product a good texture and, when the product is made into a paste form by admixture of water, it promotes easy application and it also conditions the hair.

Although the invention relates to dyeing and cosmetic products generally, a description will be given in relation to a hair dye.

One main component of the product is henna, which is a colouring agent and which adds shine to the hair. Herbs which can add colour to the hair, e.g. chamomile, can be added to the mixture and also herbs which condition only.

Cocoa butter is crucial to forming a stable solid product which promotes easy application and gives condition to the hair. An unacceptably substandard product would result if this component were wholly replaced; but other natural waxes, butters, synthetic waxes and the like can be used as partial replacements.

A mixture of 70% henna and 30% cocoa butter (by weight) formes a paste that hardens over a period of a few hours. The resultant solid material can be cut and small pieces can be broken off to suit specific requirements. The material and its easy application is thus in stark contrast to the usual form for a henna to be packaged in, this being a powder.

One example of the relative ratios of the components is given above. These can of course be varied, the requirements being only that a useable solid form product results. It appears most preferable for the henna to constitute 60% to 80% of the initial mixture and cocoa butter 20 % to 40%. Stated generally, the broad range is for the henna to constitute 40% to 90% and the cocoa butter 10% to 60%.

Small amounts of additives can be included, such as surfactants, conditioning agents, fragrances, herbs, fruit extracts, vegetables and the like. Other beneficial additives can include other natural waxes or butters, synthetic waxes, surfactants, humectants, for example glycerine, herbs in powder or whole form, vegetable oils, and the like. Further, a colouring additive may be used to provide lettering or the like in the product.

Although the above description has been of a solid dyeing cosmetic product, it will be appreciated that the invention is equally well suited to a product suitable for dyeing many materials, for example, leather.

## Claims

1. A product comprising as main components henna and cocoa butter, the product having a solid form and being prepared by mixing henna and cocoa butter to form a paste, moulding the mixture in a mould and allowing the paste to harden.

2. A product as claimed in claim 1 prepared from a mixture including 40% to 90% by weight of henna.

3. A product as claimed in claim 2 prepared from a mixture including 60% to 80% by weight of henna.

4. A product as claimed in claim 1 prepared from a mixture including 10% to 60% by weight of cocoa butter.

5. A product as claimed in claim 3 prepared from a mixture including 20% to 40% by weight of cocoa butter,

6. A product as claimed in claim 1 prepared from a mixture including 70% by weight of henna and 30% by weight of cocoa butter.

7. A product was claimed in any of claims 1 to 5 and further including one or more additives.

8. A product as claimed in claim 7 wherein the additive is one or more of a surfactant, a conditioning agent, a fragrance, a herb, a fruit extract, a vegetable.

9. A product as claimed in claim 7 wherein the additive is one or more of a natural wax or butter, a synthetic wax, a humectant, glycerine, a herb in powder or whole form, a vegetable oil.

10. A product as claimed in claim 7 including a fragrance as an additive.

11. A product as claimed in claim 7 including a fruit juice as an additive.

12. A product as claimed in any preceding claim wherein the product has a surface imprint.

13. A product as claimed in any preceding claim and further including a colouring additive providing lettering or the like in the product.

14. A product as claimed in any preceding claim, wherein the product is a cosmetic product.

15. A method of forming a solid form product comprising the step of mixing henna and cocoa butter to form a past, moulding the mixture in a mould and allowing the paste to harden.

## Patentansprüche

1. Produkt, umfassend als Hauptkomponenten Henna und Kakaobutter, wobei das Produkt eine feste Form aufweist und durch Mischen von Henna und Kakaobutter zur Bildung einer Paste, Formen des Gemisches in einer Form, und Härtenlassen der Paste hergestellt ist.

2. Produkt nach Anspruch 1, das aus einem Gemisch hergestellt ist, das 40 bis 90 Gewichtsprozent Henna enthält.

3. Produkt nach Anspruch 2, das aus einem Gemisch hergestellt ist, das 60 bis 80 Gewichtsprozent Henna enthält.

4. Produkt nach Anspruch 1, das aus einem Gemisch hergestellt ist, das 10 bis 60 Gewichtsprozent Kakaobutter enthält.

5. Produkt nach Anspruch 3, das aus einem Gemisch hergestellt ist, das 20 bis 40 Gewichtsprozent Kakaobutter enthält.

6. Produkt nach Anspruch 1, das aus einem Gemisch hergestellt ist, das 70 Gewichtsprozent Henna und 30 Gewichtsprozent Kakaobutter enthält.

7. Produkt nach einem der Ansprüche 1 bis 5 und des Weiteren enthaltend einen oder mehrere Zusatzstoffe.

8. Produkt nach Anspruch 8, wobei der Zusatzstoff eines oder mehr von einer oberflächenaktiven Substanz, einem Konditioniermittel, einem Duftstoff, einem Heilkraut, einem Fruchtextrakt, einer Pflanze ist.

9. Produkt nach Anspruch 7, wobei der Zusatzstoff eines oder mehr von einem natürlichen Wachs oder Butter, einem synthetischem Wachs, einem Feuchthaltemittel, Glycerin, einem Heilkraut in Pulverform oder ganzer Form, einem pflanzlichen Öl ist.

10. Produkt nach Anspruch 7, das einen Duftstoff als Zusatzstoff enthält.

11. Produkt nach Anspruch 7, das einen Fruchtsaft als Zusatzstoff enthält.

12. Produkt nach einem der vorangehenden Ansprüche, wobei das Produkt eine Oberflächenprägung aufweist.

13. Produkt nach einem der vorangehenden Ansprüche und des Weiteren enthaltend einen färbenden Zusatzstoff, der Buchstaben oder dergleichen in dem Produkt bereitstellt.

14. Produkt nach einem der vorangehenden Ansprüche, wobei das Produkt ein kosmetisches Produkt ist.

15. Verfahren zur Bildung eines Produkts in fester Form, umfassend den Schritt zum Mischen von Henna und Kakaobutter zur Bildung einer Paste, Formen des Gemisches in einer Form, und Härtenlassen der Paste.

## Revendications

1. Produit comprenant, en tant que composants principaux, du henné et du beurre de cacao, le produit ayant une forme solide et étant préparé en mélangeant le henné et le beurre de cacao pour former une pâte, en moulant le mélange dans un moule, et en laissant durcir la pâte.

2. Produit selon la revendication 1, préparé à partir d'un mélange comprenant de 40 % à 90 % en poids de henné.

3. Produit selon la revendication 2, préparé à partir d'un mélange comprenant de 60 % à 80 % en poids de henné.

4. Produit selon la revendication 1, préparé à partir d'un mélange comprenant de 10 % à 60 % en poids de beurre de cacao.

5. Produit selon la revendication 3, préparé à partir d'un mélange comprenant de 20 % à 40 % en poids de beurre de cacao.

6. Produit selon la revendication 1, préparé à partir d'un mélange comprenant 70 % en poids de henné et 30 % en poids de beurre de cacao.

7. Produit selon l'une quelconque des revendications 1 à 5, comprenant en outre un ou plusieurs additifs.

8. Produit selon la revendication 7, dans lequel l'additif est un ou plusieurs d'un surfactant, d'un agent conditionneur, d'un parfum, d'une plante herbacée, d'un extrait de fruit, d'un légume.

9. Produit selon la revendication 7, dans lequel l'additif est un ou plusieurs d'une cire naturelle ou d'un beurre naturel, d'une cire synthétique, d'un humectant, de la glycérine, d'une plante herbacée sous une forme pulvérulente ou entière, d'une huile végétale.

10. Produit selon la revendication 7, comprenant une fragrance en tant qu'un additif.

11. Produit selon la revendication 7, comprenant un jus de fruit en tant qu'un additif.

12. Produit selon l'une quelconque des revendications précédentes, le produit présentant une empreinte sur sa surface.

13. Produit selon l'une quelconque des revendications précédentes, comprenant en outre un additif colorant formant des lettres ou équivalents dans le produit.

14. Produit selon l'une quelconque des revendications précédentes, le produit étant un produit cosmétique.

15. Procédé de formation d'un produit solide qui comprend l'étape consistant à mélanger du henné et du beurre de cacao pour former une pâte, à mouler le mélange dans un moule, et à laisser durcir la pâte.
